# EUROPEAN PATENT SPECIFICATION

(11) **EP 4 165 401 B1**
(45) Date of publication and mention of the grant of the patent: **08.07.2026**
(21) Application number: 21822414.5
(22) Date of filing: 10.06.2021
(51) Int. Cl.: G01N 27/327, B01L 3/00

(54) **APPARATUSES AND METHODS FOR DETECTING INFECTIOUS DISEASE AGENTS**
VORRICHTUNGEN UND VERFAHREN ZUM NACHWEIS VON INFEKTIONSKRANKHEITSMITTELN
APPAREILS ET MÉTHODES DE DÉTECTION D'AGENTS DE MALADIES INFECTIEUSES

(30) Priority: 11.06.2020 US 202063038115 P
(43) Date of publication of application: 19.04.2023
(73) Proprietor: Cardiai Technologies Ltd., Calgary, AB T2N 3V6 (CA)
(72) Inventor: KOUL, Raman, Calgary, Alberta T2K 4Z3 (CA); BHAT, Sumrita, Calgary, Alberta T2K 4Z3 (CA); VASTAREY, Nikhil Suresh, Calgary, Alberta T2K 2E9 (CA); KAPOOR, Anmol Singh, Calgary, Alberta T2N 3V6 (CA)
(74) Representative: Nederlandsch Octrooibureau
(86) International application number: PCT/CA2021/050794
(87) International publication number: WO 2021/248246

(56) References cited:
- WO-A1-2020/087187
- US-A1- 2008 297 169
- US-A1- 2009 166 196
- US-B2- 8 093 667

## Description

### CROSS-REFERENCE TO RELATED APPLICATIONS

This application claims the benefit of US Provisional Patent Application Serial No. 63/038,115 filed June 11, 2020.

### FIELD OF THE DISCLOSURE

The present disclosure relates generally to apparatuses and methods for detecting analytes, and in particular to a portable three-component electrochemical sensor system for detection of infectious disease agents and/or analytes present in bodily fluids, and a method therefor.

### BACKGROUND

Infectious diseases originating from causative agents (also called "infectious agents" or "infectious vectors") such as bacteria and/or viruses can lead to acute and chronic infections in animals and humans thereby often posing a huge risk to overall human and animal health. Such infectious diseases include but not limited to diseases related to respiratory systems, digestive systems, circulatory systems, and nervous systems originating from infectious agent/vector. An example of such infectious diseases is flu (also commonly referred to as influenza) caused by one of several related "RNA viruses" (i.e., viruses whose genetic material is RNA) of the Orthomyxoviridae family, and characterized by fever, headache, fatigue, and other symptoms.

Other examples include sever acute respiratory syndrome (SARS), Middle East respiratory syndrome (MERS-CoV), novel coronavirus (2019-nCoV or SARS-CoV-2) which caused the COVID-19 pandemic; hereinafter, the terms "coronavirus", "SARS-CoV-2" and "COVID-19" may be used interchangeably), hepatitis, plague, and the like. Some of the infectious diseases are highly contagious and may lead to an epidemic which may spread quickly across continents and then to a pandemic over the entire world. Such pandemic events have huge impacts on societies globally.

A historical example of a pandemic is the "bubonic plague" which killed millions of peoples across the world. In 2003, the SARS outbreak killed at least 1,000 people, infected about 8,000 people across the Asia-Pacific region, and caused disruption in travel and closure of workplaces with an overall economic loss of about $40 billion. While some vaccines have become available for limiting the spread of specific infectious diseases, rapid detection and diagnosis are generally the keys to stop the spread of an infectious disease.

US Patent Application Publication No. 2008/0297169 A1 to Greenquist, et al. discloses a device, test cards, methods and kits which are useful for determining the particle fraction and rate of viscosity of a fluid sample, the presence of an analyte in a fluid sample, or the aggregation of particles in a fluid sample to detect an analyte or as an immunologic assay.

US Patent Application Publication No. 2009/0166196 A1 to Kayyem discloses methods and apparatus for conducting analyses, particularly microfluidic devices for the detection of target analytes.

Therefore, it is highly desirable for improved, efficient, and rapid methods for the detecting and identifying infectious agents that cause diseases such as influenza, respiratory diseases, SARS-Cov-2, sexually transmitted diseases, blood diseases, viral diseases, bacterial diseases, and/or the like. Moreover, there exists a need of rapid and quantitative serological assays with portability, ultra-sensitivity, and reasonable throughput for detection of COVID-19 in order to timely diagnose the disease.

### SUMMARY

The present invention provides a portable electrochemical-sensor system for detection of infectious disease agents and a method of use therefor, as defined by the appended independent claims. The system allows a rapid detection of infection/infectious disease via one or more detection routes, simultaneously from varied biological samples such as cells, tissues, bodily fluids, and/or the like.

The system comprises an analyte processing and presentation device for collecting samples and presenting collected samples to an electrochemical-sensor structure or electrochemical biosensor which may be in the form of a strip, and a diagnostic bio-sensing apparatus or electrochemical-based detection structure for capturing and analyzing infectious agent, biproducts, biomolecule, biomarker, nucleic acids (targets), and/or the like.

Samples are applied through a sample port of the microfluidic device which collects and prepares samples received from the sample port and delivers the collected and prepared samples to the sensing structure for further analysis. The delivered prepped sample is analyzed via electrochemical-based methods to give a corresponding readable output when integrated with an electronic reader.

In some embodiments, an electronic reader contains embedded components and analog/digital circuitry. The electronic reader may contain an LCD screen for user interfacing and may contain communication modules to transmit data to a computing device. The electronic reader may also comprise inlet ports to communicate with sample-preparation device.

In some embodiments, the system may also comprise a combination of test kits, software application programs, App, servers, and websites. The bio-sensing technology disclosed herein may simultaneously perform (1) detections of an infectious disease agent, vector, and/or the like and (2) monitoring of immunological response to the infectious agent, in order to detect, monitor, and limit spread of contagious disease. The application programs may manage the data transfer from the PoC device to a server for:
1) data collection,
2) communication with a Health Care Organization (HCO),
3) population screening, and
4) remote feedback to patients with information pertaining to their health status.

In some embodiments, the analyte processing and presentation device may be a biochip for sample processing and presentation.

In some embodiments, the analyte processing and presentation device may be a microcapillary device for sample processing and presentation.

In some embodiments, sample collection devices in conjunction with the analyte processing and presentation device may be used to process and present the sample to the electrochemical-sensor structure.

In some embodiments, the analyte processing and presentation device may be a microcolumn to present sample/analyte to the electrochemical-sensor structure.

In some embodiments, the analyte processing and presentation device may be a lateral-flow device to present sample/analyte to the electrochemical-sensor structure.

In some embodiments, the analyte processing and presentation device may be a flow-cell device to present sample/analyte to the electrochemical-sensor structure.

In between its inlet and outlet ports, multiple chambers are embossed along the flow path.

In some embodiments, the chambers may be designed to limit flow rate of fluid.

In some embodiments, the chambers may contain embossed or debossed structure to promote capillary flow.

In some embodiments, the chambers may contain embossed or debossed structures to enable mixing between two or more molecular, oligonucleotide, enzymatic, or protein entities with the fluid.

In some embodiments, the chamber may contain conductive substrates to detect impedimetric changes per the nature of the fluid.

In some embodiments, the chamber may contain optically active components wherein the change in its absorptive properties can be correlated to, but not limited to, its volume, viscosity, reflectance, and fluoro-emissive properties.

In some embodiments, the flow cell may be used in congruence with another flow cell where each flow cell prepares and presents sample with varied degree of dilution.

In some embodiments, the flow cell may be used in congruence with another flow cell of similar design to prepare and present samples to a common or multiple outlet ports, where each sample has been separately subjected to react, mix, bind, or entrap with molecules, enzymes, oligonucleotides and antibodies within its corresponding flow paths.

In some embodiments, the system may comprise a multi-working electrode sensing system embedded with zinc oxide (ZnO) arranged to immobilize or otherwise capture DNA, aptamer, and protein probes, for the detection of target infectious agents such as analytes from bodily fluids, and a multichannel potentiostat system with integrated BLUETOOTH^{®} data transfer system and microcontroller to analyze the electrochemical signals and calculate concentration of the target infectious agents.

In some embodiments, the control electrode of the multi-working electrode sensing system is a saturated electrode that may signal the matrix properties while the other working electrodes may detect one or more infectious target nucleic acids.

In some embodiments, the microcontroller may be connected to a custom analog front end that measures electrochemical parameters.

In some embodiments, the electrochemical parameters may include electrochemical and amperometry measurements of the substrate.

In some embodiments, the electrochemical measurement circuit may be replicated as per the number of strips being used in the platform.

In some embodiments, the electrochemical measurement circuit may be replicated as per the number of active areas that may contain one or more working electrodes.

In some embodiments, the bio-sensing apparatus may be connected to Apps, software, server, and website to monitor patients immunological response (via immune markers) in order to detect, monitor, and limit spread of contagious disease. The App may manage the data transfer from the kits to the server for data collection, communication with HCO (or other central health systems), population screening, and may provide remote feedback to patients regarding their health status.

In some embodiments, the apparatus may manage patient data.

In some embodiments, the apparatus may involve means of communicating data to the local health authority.

In some embodiments, the apparatus may have a central server or multiple servers for storing patient information.

In some embodiments, the apparatus may have a website that communicates with the server to present patient information in forms of graphs and profiles.

In some embodiments, the apparatus may aggregate data to segregate data based on population, ethnicity, age and patient history.

In some embodiments, the apparatus may enable an App to communicate with a patient regarding a multiple set of tests.

In some embodiments, the apparatus may convey health and diagnostic results to a patient for guidance to a next step by offering the first set of tests.

In some embodiments, the patient may be led, by means of communications from an App, to proceed to a second set of tests or to consult with their health care professional.

In some embodiments, the device may have a heating component/element/substance, with an adhered heat sink; wherein the heat sink may be a liquid exchange component, a heat paste, or a network of microfluidic channels to enable heat exchange.

In some embodiments, the electrochemical sensing structure further comprises an identification circuitry separated from the first circuitry for indicating one or more biomarkers of the bodily fluid sample analyzable using the electrochemical-sensor structure.

In some embodiments, the electrochemical sensing structure is a testing strip.

In some embodiments, the analyte processing and presentation apparatus is a microcolumn device or a lateral-flow device.

In some embodiments, the analyte processing and presentation apparatus is a flow-cell device comprising one or more flow cells; each of the one or more flow cells comprises: an inlet for receiving the bodily fluid sample from the first port, an outlet in the second port, and a flow path for delivering the bodily fluid sample from the inlet to the outlet; and the flow path comprises a plurality of chambers embossed therealong.

In some embodiments, the plurality of chambers are configured for limiting a flow rate of the bodily fluid sample in the flow path.

In some embodiments, a first set of one or more of the plurality of chambers comprise a first type of embossed or debossed structures for promoting capillary flow.

In some embodiments, a second set of one or more of the plurality of chambers comprise embossed or debossed structures for mixing between two or more molecular, oligonucleotide, enzymatic, or protein entities with the bodily fluid sample.

In some embodiments, a third set of one or more of the plurality of chambers comprise conductive substrates for detecting impedimetric changes caused by the bodily fluid sample.

In some embodiments, a fourth set of one or more of the plurality of chambers comprise optically active components with changeable absorptive properties correlated to the volume, viscosity, reflectance, and/or fluoro-emissive properties of the bodily fluid sample.

In some embodiments, the analyte processing and presentation apparatus comprises a plurality of flow cells.

In some embodiments, each flow cell is configured for preparing and presenting the bodily fluid sample with a different degree of dilution.

In some embodiments, each flow cell is configured for preparing and presenting a portion of the bodily fluid sample to the outlet thereof; and said preparing and presenting the portion of the bodily fluid sample comprises reacting, mixing, binding, or entrapping the bodily fluid sample with molecules, enzymes, oligonucleotides, and/or antibodies within the flow path of the flow cell.

In some embodiments, the system further comprises one or more sample collection devices for using with the analyte processing and presentation apparatus for processing and presenting the bodily fluid sample to the electrochemical-sensor structure.

In some embodiments, the method further comprises: removing the electrochemical sensing structure from the analyte processing and presentation apparatus.

### BRIEF DESCRIPTION OF THE DRAWINGS

FIG. 1 is a schematic perspective view of a portable electrochemical-sensor system, according to some embodiments of this disclosure, the portable electrochemical-sensor system comprising a portable point-of-care (PoC) device, an electrochemical-sensor structure, and an analyte processing and presentation device;
FIG. 1B is a schematic plan view of the electrochemical-sensor system shown in FIG. 1A;
FIG. 2 is a schematic plan view of the electrochemical-sensor structure of the electrochemical-sensor system shown in FIG. 1A, the electrochemical-sensor structure comprising a plurality of electrodes;
FIGs. 3A and 3B are schematic diagrams of the circuitries of the PoC device shown in FIG. 1A for electrically coupling to the electrodes of the electrochemical-sensor structure shown in FIG. 2 for measuring one or more biomarkers in the samples on the electrochemical-sensor structure;
FIG. 4 is a schematic view of the electrochemical-sensor structure shown in FIG. 2, illustrating the substrate and the WE, wherein the WE comprises a nanostructured-sensing surface embedded with ZnO;
FIG. 5 is a schematic cross-section view of the analyte processing and presentation device of the electrochemical-sensor system shown in FIG. 1A, according to some embodiments of this disclosure;
FIG. 6 is a flowchart showing a process for using the portable electrochemical-sensor system shown in FIG. 1A to determine infection of an infectious disease/analyte;
FIG. 7 is a schematic diagram of a personalized health-monitoring system, according to some embodiments of the present disclosure;
FIG. 8 is a schematic diagram showing a simplified hardware structure of a computing device of the personalized health-monitoring system shown in FIG. 7;
FIG. 9 is a schematic diagram showing a simplified software architecture of a computing device of the personalized health-monitoring system shown in FIG. 7; and
FIG. 10 is a block diagram showing a functional structure of the personalized health-monitoring system shown in FIG. 7.

### DETAILED DESCRIPTION

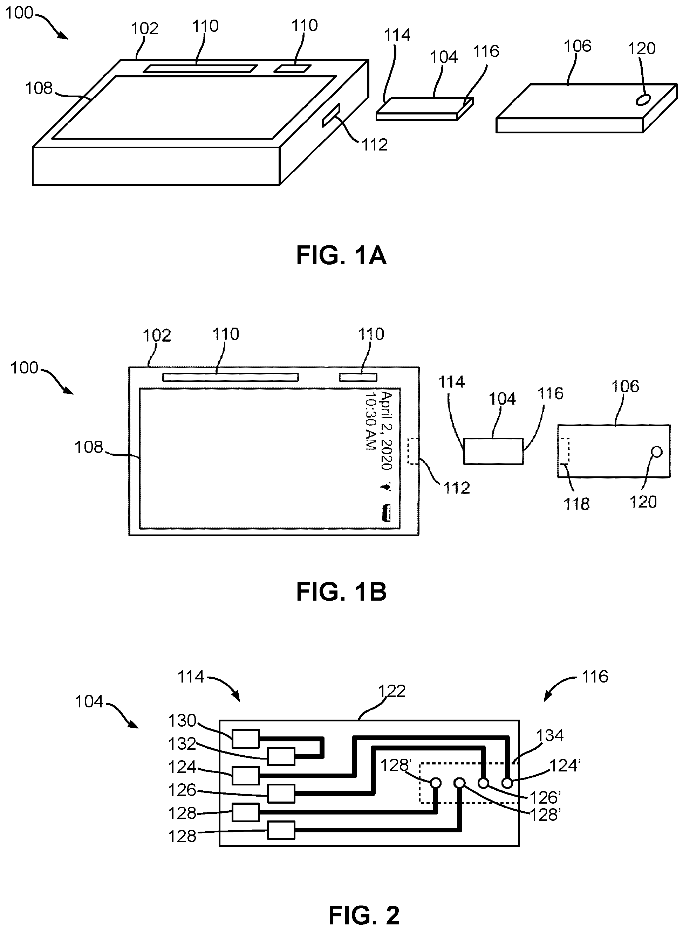

Turning now to FIGs. 1A and 1B, a portable electrochemical-sensor system is shown and is generally identified using the reference numeral 100, used for analyzing, determining, and monitoring a user's health conditions, including analytes or infection by an infectious disease such as SARS, MERS-CoV, SARS-CoV-2, and/or the like.

The portable electrochemical-sensor system may be used for home-based testing for disease diagnosis and prognosis. However, those skilled in the art will appreciate that the portable electrochemical-sensor system 100 may also be used in other suitable places such as health centers, clinics, hospital, and the like.

As shown in FIGs. 1A and 1B, the portable electrochemical-sensor system 100 in these embodiments comprises a portable diagnostic bio-sensing apparatus 102 (also denoted an "electrochemical-sensing apparatus") in the form of a point-of-care (PoC) device with a size suitable for personal use, a disposable electrochemical-sensor structure 104 in the form of a strip, and an analyte processing and presentation device 106 in the form a microfluidic device or a flow-cell device, for analyzing, determining, and monitoring user's health conditions by detecting various analyte in the patient's biological samples such as bodily fluid samples received onto the electrochemical-sensor structure. The detection of the analyte is used for detecting infectious agents and assessing the patient's health conditions with respect to an infectious disease, wherein the presence, absence, or variation in the quantities of a certain analyte the biological samples may be used as an indicator or predictor of disease. Herein, infectious agents or infectious vectors may be nucleic acids, blood-born vectors, zoonotic diseases, microbes (such as bacteria, viruses, fungi, protozoa, and/or the like), helminths, host immunoglobulins, and/or the like.

The portable diagnostic apparatus 102 and the electrochemical-sensor structure 104 may be similar to those disclosed in Applicant's Canadian Patent Application Ser. No. 3,060,849, entitled "PORTABLE ELECTROCHEMICAL-SENSOR SYSTEM FOR ANALYZING USER HEALTH CONDITIONS AND METHOD THEREOF", filed on Nov. 04, 2019.

In particular, the PoC device 102 in these embodiments comprises a screen 108, a user-input structure for receiving user inputs, a strip-receiving port 112 for receiving a proximal side 114 of the electrochemical-sensor structure 104, a control circuitry having a control structure (not shown) such as a RFduino microcontroller offered by RFduino Inc. of Hermosa Beach, CA, USA, and relevant circuitries. The PoC device 102 also comprises a power source such as battery for powering various components.

The user-input structure may comprise one or more buttons 110 and/or a touch-sensitive screen (such as a touch-sensitive screen 108 in some embodiments) for receiving user inputs such as user instructions (e.g., turning the PoC device 102 on or off, starting a diagnostic process, displaying readings obtained in the diagnostic process, displaying previous diagnostic readings, and/or the like) and/or user data (e.g., the user's age, sex, weight, height, and/or the like).

The control circuitry includes an analysis circuitry such as a potentiostat circuitry for bio-sensing (described in more detail later) and a monitoring circuitry for other tasks such as performing user-instructed operations, detecting the insertion of the electrochemical-sensor structure 104, reading and displaying the measured levels of biomarkers, storing measurement data, transmitting measurement data to a remote device for trend tracking, and/or the like. In various embodiments, the analysis circuitry and monitoring circuitry may use the same microcontroller or alternatively use separate microcontrollers.

As shown in FIG. 2, the electrochemical-sensor structure 104 comprises a plurality of electrodes 124 to 132 distributed on a biocompatible substrate 122 that enables fluid to flow thereon.

In particular, the electrochemical-sensor structure 104 in this embodiment comprises a reference electrode (RE) 124, a control electrode (CE) 126, and two working electrodes (WEs) 128 on the proximal side 114 of thereof and extending into a sampling region 134 (also called a sample-receiving region) adjacent a distal side 116 thereof and forming corresponding RE 124', CE 126' and WEs 128', for measuring the electrochemical properties of samples (not shown) received therein.

In these embodiments, the surfaces of the WEs 128' may be modified or otherwise treated with a mediator to mediate the electron transfer from the electrodes to body fluids. Different WEs 128' may be configured to harbor different elements for detecting one or more analyte, agents, and/or targets.

The electrochemical-sensor structure 104 also comprises a pair of identification electrodes 130 and 132 on the proximal side 114 thereof, joined by a trace with a pre-defined resistance or a pre-defined impedance, for indicating the type of biomarker or infectious agent that the electrochemical-sensor structure 104 is suitable to detect. The elements from 124 to 132 may be made of or comprise conductive or semi-conductive metals such as gold (Au), chromium (Cr), titanium, platinum, silver, and/or the like.

As described above, the analysis circuitry of the portable diagnostic bio-sensing apparatus 102 is designed to correspond with the circuitry of the electrochemical-sensor structure 104 for monitoring the electrochemical reaction between the analyte in the samples and the detection elements on the electrochemical-sensor structure 104. With the electrochemical-sensor structure 104 shown in FIG. 2, the analysis circuitry of the portable diagnostic bio-sensing apparatus 102 correspondingly comprises a set of coupling electrodes in the strip-receiving port 112 for electrically engaging the electrodes 124 to 132 of the electrochemical-sensor structure 104.

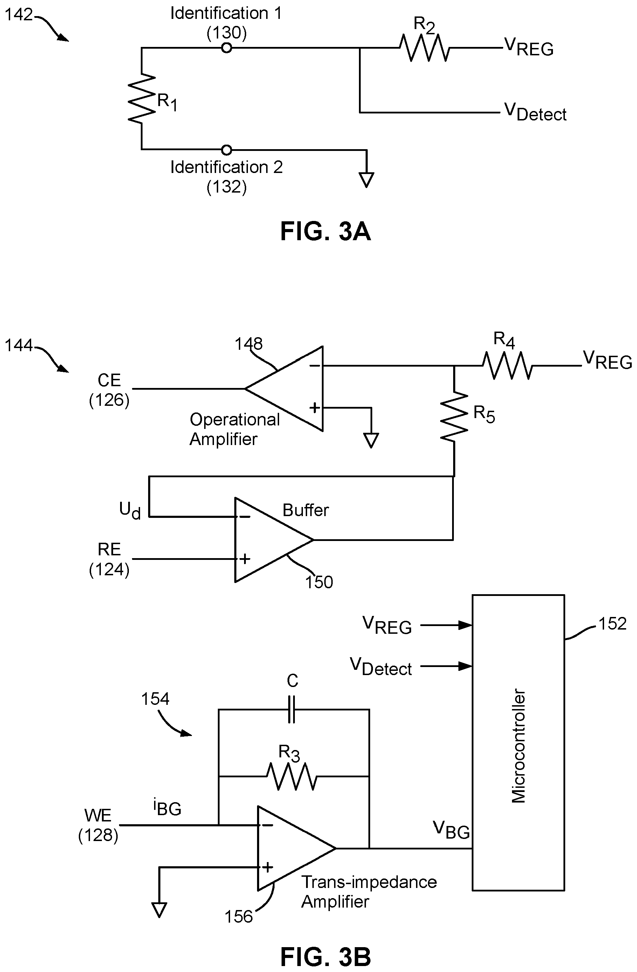

As shown in FIGs. 3A and 3B, the PoC device 102 comprises a plurality of circuitries 142 and 144 for electrically engaging the electrodes 124 to 132 when the proximal side 114 of the electrochemical-sensor structure 104 is inserted into the strip-receiving port 112.

As shown in FIG. 3A, a first circuitry 142 in the form of a voltage divider is used for determining the type of the biomarker. As described above, the identification electrodes 130 and 132 have a pre-defined resistance therebetween which is represented by a resistor R₁. The resistance of R₁ is predefined and indicative of the type of biomarker that the electrochemical-sensor structure 104 is suitable to detect. The second circuitry 144 electrically engages the identification electrodes 130 and 132 and applies a voltage V_{REG} (e.g., 3.3V) thereto via a resistor R₂ with known resistance. A voltage signal V_{Detect} is outputted from between R₁ and R₂. Therefore, V_{Detect} = V_{REG} R₁/(R₁+R₂), and the resistance of R₁ and in turn the type of the biomarker may be obtained by comparing V_{Detect} with V_{REG}.

As shown in FIG. 3B, a second circuitry 144 in the form of a Direct-Current (DC) potentiostat circuitry is used to control the voltage between the WE 128 and RE 124.

In these embodiments, the samples on the electrochemical-sensor structure 104 act as the electrolyte between the WE 128 (acting as a cathode), the RE 124 (acting as an anode), and the CE 126. Because of the nature of the operational amplifier 148, a current is supplied through the CE 126 until the voltage at RE 124 and U_{d} is the same. Thus, U_{d} determines the voltage of the electrolyte, and consequently determines the accuracy of biomarker measurements as a too-low U_{d} may not be able to generate a sufficient measurement resolution and a too-high U_{d} may trigger inferencing reactions or surface property changes.

Thus, in the circuitry 144, the three-electrode configuration is connected to a DC potentiostat circuitry wherein a constant DC voltage is regulated and applied over the WE 128 and RE 124 of the electrochemical-sensor structure 104. The circuitry 144 may be used for determining the electrochemical properties of a bodily fluid sample for analysis of the bodily fluid sample by detecting and determining impedimetric measurements.

As shown in FIG. 3B, a control structure 152 (e.g., a microcontroller) compares V_{Detect} with V_{REG} and determines the type of the biomarker. The microcontroller 152 then adjusts the bio-sensing parameters (such as U_{d}) to adapt to the determined type of the biomarker and measures the voltage of WE 128. An amplifying circuitry 154 which in this embodiment comprises an amplifier 156, a resistor R₃, and a capacitor C is used to amplify the signal of WE 128. In this way, the electrochemical properties of the biomarker in the samples on the electrochemical-sensor structure 104 are measured and are used for determining the patient's health conditions.

In some embodiments, the DC potentiostat circuitry may be coupled to a frequency-response analyzer used as an impedance-analysis system. For example, the system may contain four stages: current-to-voltage conversion with a multiplexer, an amplifier to accommodate extra electrodes within the system, a gain stage, and an eventual frequency-response analyzer implemented as an integrated circuit (IC). In various embodiments, the multiplexer may be used to switch the system between a calibration mode and one or more multiple-electrode modes.

Those skilled in the art will appreciate that the circuitry 144 may also be used for amperometric types of measurements which are commonly used for glucose detection. Moreover, the potentiometric type of measurements may also be implemented using the three-electrode configuration, wherein an Alternate-Current (AC) wave with a predefined frequency is applied for stimulating the samples while forward (e.g. by increasing the voltage) and reverse (e.g. by decreasing the voltage) current is measured to yield a differential current (forward-reverse).

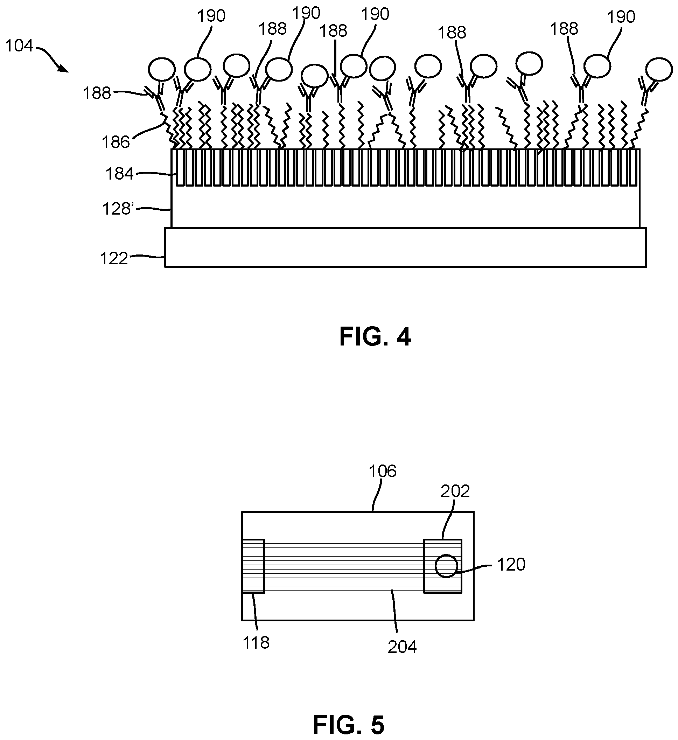

FIG. 4 is a schematic view of the electrochemical-sensor structure 104 showing the substrate 122 and the electrode WE 128'. As shown, the electrochemical-sensor structure 104 comprises a nanostructured-sensing surface in the sampling region 134 thereof for amplifying the amount of biomarker binding to the electrochemical-sensor structure 104 in order to achieve improved sensitivity.

In particular, the distal-side electrode WE 128' comprises a nanostructured-sensing surface 182 having a plurality of capture areas184 with zinc oxide (ZnO) nanomaterials. In some embodiments, the ZnO nanomaterials may be embedded into the detection areas. In some other embodiments, the ZnO nanomaterials may be synthesized by depositing ZnO onto the distal-side electrode WE 128' on the substrate (acting as seeds) and then immersing the substrate consisting the coated electrode in a chemical bath consisting of zinc nitrate hexahydrate and hexamethyline tetramine at a temperature below the boiling point of water and preferably about 80°C for "growing" the ZnO nanomaterials.

The capture areas 184 are coated with a specific type of detection element 188 such as one or more immobilized capture ligand such as antibodies, enzymes, nucleic acid aptamers, and the like, for detecting a specific biomarker 190 for which the detection element 188 has a high specificity and affinity. The capture areas 184 are also coated with crosslinking molecules 186 which immobilize the detection-element molecules 188 onto the capture areas 184 for capturing and reacting with the corresponding biomarkers 190.

When samples are received into the sampling region 134 of the electrochemical-sensor structure 104, electrochemical interaction between the analyte in the samples and the detection elements occur and cause the electrochemical changes. The electrochemical-sensor structure 104 is engaged with an ex-vivo PoC device 102 which imparts energy to the biological samples and measures the electrochemical properties thereof for generating a reading indicative of the concentration of a specific compound in the bodily fluid sample. The imparted energy may be electrical energy and the measured energy property may be the potential difference, current, impedance, and/or the like.

Referring to FIG. 5 and also to FIGs. 1A and 1B, the microfluidic device 106 comprises a strip-receiving port 118 (also called a "coupling port") for receiving the distal side 116 of the electrochemical-sensor structure 104 and an applicator-receiving port 120 (also called a "sampling port") for receiving a sampling head of an applicator such as a swab. In these embodiments, the strip-receiving port 118 and the applicator-receiving port 120 are arranged on opposite sides of the microfluidic device 106 along a longitudinal axis thereof. The microfluidic device 106 may comprise an analyte processing and presentation structure 202 in fluid communication with the applicator-receiving port 120 for receiving bodily fluid sample from the applicator-receiving port 120 and processing the bodily fluid sample so that the analyte in the bodily fluid sample is prepared (e.g., with suitable concentration) for the PoC device 102 to analyze. The applicator-receiving port 120 or more specifically the analyte processing and presentation structure 202 is in fluid communication with the strip-receiving port 118 via one or more microfluidic channels 204 for delivering the bodily fluid sample to the strip-receiving port 118.

The microfluidic device 106 may be any suitable device with a microfluidic structure for samples collection and presentation. For example, in some embodiments, the microfluidic device 106 may be a microcapillary device for sample processing and presentation.

In some embodiments, the microfluidic device 106 may be a microcolumn device to present sample/analyte to the electrochemical-sensor structure.

In some embodiments, the microfluidic device 106 may be a lateral-flow device to present sample/analyte to the electrochemical-sensor structure.

In some embodiments, the microfluidic device 106 may be a flow-cell device to present sample/analyte to the electrochemical-sensor structure comprising one or more miniaturized flow cells.

In some embodiments, the miniaturized flow cell may contain an inlet in fluid communication with an outlet port through a flow path. In between the inlet and outlet ports, a plurality of chambers are embossed along the flow path. In some embodiments, the chambers may be designed to limit flow rate of the bodily fluid sample.

In some embodiments, the chambers may contain a first type of embossed or debossed structure to promote capillary flow.

In some embodiments, the chambers may contain a second type of embossed or debossed structures to enable mixing between two or more molecular, oligonucleotide, enzymatic, or protein entities with the bodily fluid sample.

In some embodiments, the chamber may contain conductive substrates to detect impedimetric changes caused by the bodily fluid sample.

In some embodiments, the chamber may contain optically active components wherein the change in their absorptive properties can be correlated to, but not limited to, its volume, viscosity, reflectance, and fluoro-emissive properties of the bodily fluid sample.

In some embodiments, the flow cell may be used in congruence with another flow cell where each flow cell prepares and presents sample with varied degree of dilution.

In some embodiments, the flow cell may be used in congruence with another flow cell of similar design to prepare and present samples to a common or multiple outlet ports, where each sample has been separately subjected to react, mix, bind, or entrap with molecules, enzymes, oligonucleotides, and/or antibodies within its corresponding flow paths.

In some embodiments, one or more sample collection devices in conjunction with the microfluidic device 106 may be used to process and present the sample to the electrochemical-sensor structure.

The following patent documents and academic papers describe some examples of microfluidic technologies and/or devices which may be used as or modified to be the microfluidic device 106 herein such that the microfluidic device 106 comprises a microfluidic structure fluidly connecting the applicator-receiving port 120 thereof with the distal side 116 of the electrochemical-sensor structure 104.

US Patent No. 7,094,354 to Pugia, et al*.* teaches a microfluidic device provides separation of particles in a liquid sample, particularly, separation of a sample of whole blood into its components for further analysis. Separation into red blood cells and plasma occurs within a few seconds after the blood sample has been transferred into a separation chamber with the application of centrifugal force of less than about five times gravity. With the application of greater force measurement of hematocrit is possible.

Academic paper entitled "An integrated microfluidic device for rapid and high-sensitivity analysis of circulating tumor cells" to Jiang, et al., published on Scientific Reports (DOI: 10.1038/srep42612) teaches a rapid and high-sensitivity approach for CTCs detection using an integrated microfluidic system, consisting of a deterministic lateral displacement (DLD) isolating structure, an automatic purifying device with CD45-labeled immunomagnetic beads and a capturing platform coated with rat-tail collagen. The authors observed high capture rate of 90%, purity of about 50% and viability of more than 90% at the high throughput of 1 mL/min by capturing green fluorescent protein (GFP)-positive cells from blood. Further capturing of CTCs from metastatic cancers patients revealed a positive capture rate of 83.3%. Furthermore, the device was compared with Cell Search system via parallel analysis of 30 cancer patients, to find no significant difference between the capture efficiency of both methods. However, the device displayed advantage in terms of time, sample volume and cost for analysis. Thus, the integrated device with sterile environment and convenient use will be a promising platform for CTCs detection with potential clinical application.

Academic paper entitled "Applications of Microfluidic Devices for Urology" to Han, et al., published on Int Neurourol J 2017;21 Suppl 1: S4-9, and accessible at https://doi.org/10.5213/inj.1734838.419, reviews microfluidics which is considered an important technology that is suitable for numerous biomedical applications, including cancer diagnosis, metastasis, drug delivery, and tissue engineering. The authors consider that, although microfluidics is still considered to be a new approach in urological research, several pioneering studies have been reported in recent years. In this paper, the authors reviewed urological research works using microfluidic devices. Microfluidic devices were used for the detection of prostate and bladder cancer and the characterization of cancer microenvironments. The potential applications of microfluidics in urinary analysis and sperm sorting were demonstrated. The use of microfluidic devices in urology research can provide high-throughput, high-precision, and low-cost analyzing platforms.

Academic paper entitled "Disposable microfluidic devices: fabrication, function, and application" to Fiorini, et al., published on BioTechniques 38:429-446 (March 2005) describes recent developments in microfluidics, with special emphasis on disposable plastic devices. The paper provides an overview of the common methods used in the fabrication of polymer microfluidic systems, including replica and injection molding, embossing, and laser ablation. Also described are the different methods by which on-chip operations-such as the pumping and valving of fluid flow, the mixing of different reagents, and the separation and detection of different chemical species-have been implemented in a microfluidic format. In this paper, a few select biotechnological applications of microfluidics are presented to illustrate both the utility of this technology and its potential for development in the future.

Japanese Patent No. 3,725,109 B2 to Teruo, et al.*,* teaches a micro-fluid device to reduce a dead volume, improve response, and easily change a flow passage according to applications such as analysis and composition. This micro-fluid device comprises pump units 11 and 12 having a first connection surface 12a, a pump mechanism MP, and flow passages 131 and 132 communicating with the pump mechanism MP and opening into the first connection surface 12a and a flow passage unit 13 having a second connection surface 13b for connecting with the first connection surface 12a so as to be brought into contact with and apart from the first connection surface 12a and flow passages 142, 143, 145, and 146 opening into the second connection surface 13b and connectable to the flow passages 131 and 132 of the pump unit 12. At least either of the material forming the first connection surface 12a and the material forming the second connection surface 13b is made of an elastic material having self-sealability.

US Patent No. 8,877,484 to Bau-madsen, et al.*,* teaches a microfluidic device comprising at least one test channel, which test channel comprises an upper test channel section with an upstream end and a sampling region at its upstream end and at least one reference channel, which reference channel comprises an upper reference channel section with an upstream end and a sampling region at its upstream end. The test channel and the reference channel comprise a merging region downstream to the upper test channel section and a common downstream channel section. The merging region and the common downstream channel section are arranged such that a reference liquid flowing from the upper reference channel section into the merging region will block a test liquid flow in the upper test channel section when the test liquid flow has not yet reached the merging section. The microfluid device may be used for detecting change of flow properties e.g. due to agglomeration, agglutination or viscosity change in a liquid preferably selected from water, urine, blood, or blood plasma.

Academic paper entitled "Microfluidic device to culture 3D in vitro Human Capillary Networks" to Moya, et al., published on Methods Mol Biol. 2014; 1202: 21-27. doi:10.1007/7651_2013_36, teaches that models that aim to recapitulate the dynamic in vivo features of the microcirculation are crucial for studying vascularization. Cells in vivo respond not only to biochemical cues (e.g., growth factor gradients) but also sense mechanical cues (e.g., interstitial flow, vessel perfusion). Integrating the response of cells, the stroma, and the circulation in a dynamic 3D setting will create an environment suitable for the exploration of many fundamental vascularization processes. The authors describe an in vivo-inspired microenvironment that is conducive to the development of perfused human capillaries.

European Patent No. 2,786,019 to Laetitia, et al.*,* teaches a microfluidic device including a first microchannel, a second microchannel, and a valve comprising at least an input port and an output port, the ports respectively connected to the first microchannel and the second microchannel, the valve designed to control a flow of a liquid along a flow direction (z) defined by the ports; wherein the valve further comprises one or more walls joining the ports and defining a hollow chamber that is wider than each of the microchannels in a direction perpendicular to the flow direction, the walls at least partly deformable along a deformation direction (-y) intersecting the flow direction, such that the walls can be given at least a first deformation state and a second deformation state, such that the liquid can be pulled along the flow direction substantially more in the second deformation state than in the first deformation state.

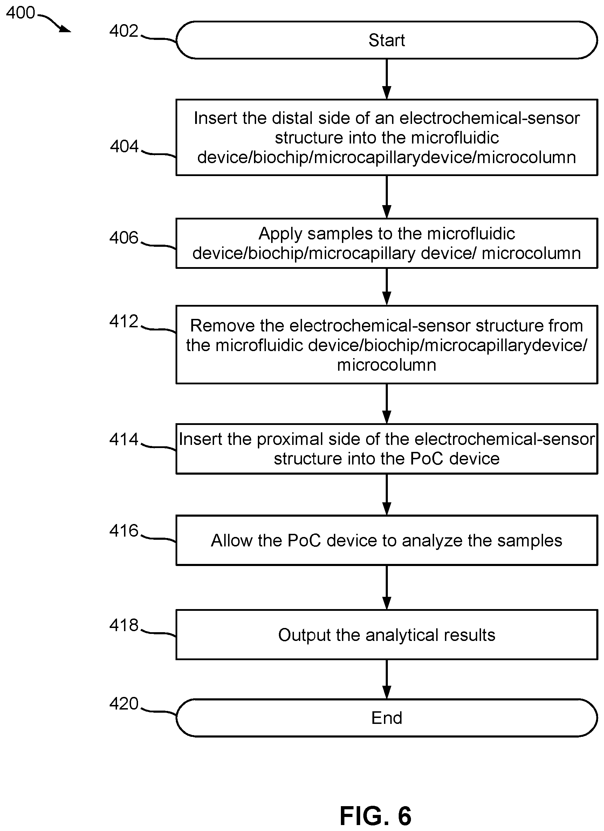

FIG. 6 is a flowchart showing a process 400 for using the portable electrochemical-sensor system 100 to determine infection of an infectious disease.

The process 400 starts (step 402) after an operator receives samples from a patient using an applicator such as a swab/or in collection tubes (not shown).

At step 404, the operator inserts the distal side 116 of an electrochemical-sensor structure 104 into the strip-receiving port 118 of the microfluidic device 106. Then, the operator applies the received samples into the microfluidic device 106 by, e.g., inserting the sampling head of the applicator into the applicator-receiving port 120 of the microfluidic device 106 (step 406). The microfluidic structure of the microfluidic device 106 then guides the samples to the distal side 116 of the electrochemical-sensor structure 104 and in contact with the electrodes (e.g., CE, RE, and WEs).

Optionally, the operator may then remove the electrochemical-sensor structure 104 from the microfluidic device 106 after samples have been presented onto the electrochemical-sensor structure 104 (step 412).

The operator then inserts the proximal side 114 of the electrochemical-sensor structure 104 into the strip-receiving port 112 of the PoC device 102 (step 414) and allows the PoC device 102 to analyze the samples (step 416).

The solution delivered to the electrodes mainly comprises analytes, nucleic acids, minimal proteins and cell content, redox probe, and/or the like. The WEs are already functionalized with capture probes for rapid detection of target genetic material, proteins, biomolecules, and/or metabolites of the infectious agent. After a sufficient period of time (e.g., about five (5) to 20 minutes) of the solution interaction with the electrodes, the PoC device 102 records the electrochemical signals and obtains the concentration of the target molecule. The entire process of sample collection from swab to electrical readout is automated without a need for any manual sample preparation, centrifuge, pipetting, aliquoting, filtration, precipitation, and elution requirements.

After analyzing the samples, the analytical results are output to a suitable target such as a display of the PoC device 102, a client-computing device and/or a server computer functionally connected to the PoC device 102 , and/or the like (step 418) and the process 400 ends (step 420). Herein, the connection between the PoC device 102 and the client-computing device and/or server computer may be established using suitable wired and/or wireless communication technologies such as Ethernet, WI-FI^{®}, (WI-FI is a registered trademark of Wi-Fi Alliance, Austin, TX, USA), BLUETOOTH^{®} (BLUETOOTH is a registered trademark of Bluetooth Sig Inc., Kirkland, WA, USA), ZIGBEE^{®} (ZIGBEE is a registered trademark of ZigBee Alliance Corp., San Ramon, CA, USA), 3G, 4G and/or 5G wireless mobile telecommunications technologies, parallel ports, serial ports, USB connections, optical connections, and/or the like.

In some embodiments, the portable electrochemical-sensor system 100 may be formed as a RNA/DNA-diagnostic kit for detecting infectious diseases.

In some embodiments, the PoC device 102 and the strip are similar to those disclosed in Applicant's Canadian Patent Application Ser. No. 3,060,849. The portable electrochemical-sensor system 100 in these embodiments may be formed as the combination of a RNA/DNA-diagnostic kit (i.e., the combination of the PoC device 102, the electrochemical-sensor structure 104, and the microfluidic device 106) and a serological kit (i.e., the combination of the PoC device 102 and the blood-testing strip) for detecting infectious diseases and for monitoring the patient's recovery therefrom.

In some embodiments, the portable electrochemical-sensor system 100 may further comprise a computerized network with one or more client-computer devices, one or more servers, and one or more other sensors, thereby forming a computerized network system for monitoring a person's health conditions. Such a network system is similar to that disclosed Applicant's PCT Patent Application Ser. No. PCT/CA2019/051244 filed September 5, 2019.

On the software side, the network system comprises one or more Apps for managing the data transfer from the portable electrochemical-sensor system 100 to the server for data collection, communication with a central healthcare system, population screening, and remote feedback to patients on their health status.

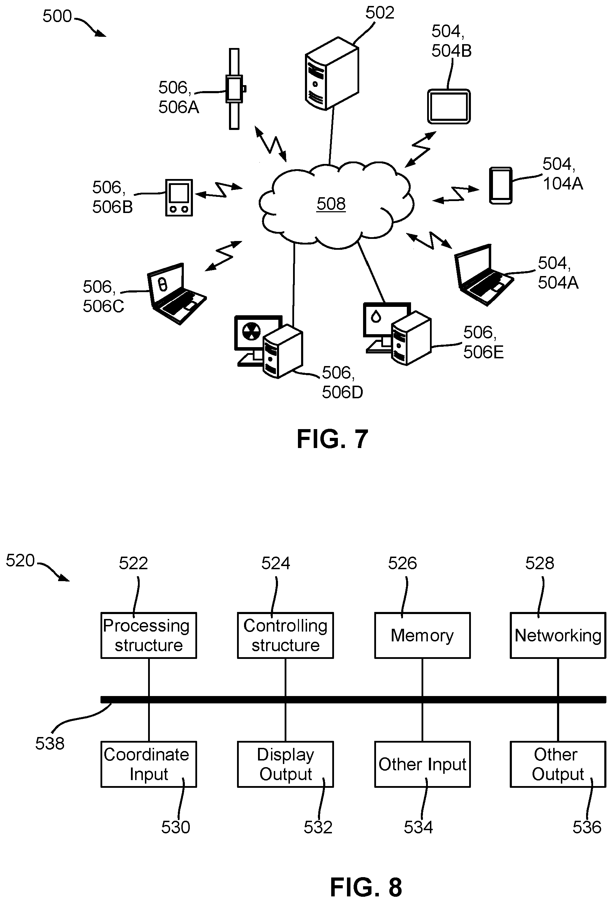

For example. FIG. 7 shows a personalized health-monitoring system 500 in some embodiments. The personalized health-monitoring system 500 has at least two types of users, including patients and doctors. Herein, the term "patient" is used for referring to a person or a user using the health-monitoring system 500 for tracking and managing his/her health conditions. Thus, the term "patient" does not necessarily imply that the person has any sickness or health issues. On the other hand, the term "doctor" refers to a user using the health-monitoring system 500 for helping the patient to track and manage the patient's health conditions.

As shown in FIG. 7, the personalized health-monitoring system 500 comprises a server computer 502, a plurality of client-computing devices 504, and one or more health-monitoring data-sources 506 functionally interconnected by a network 508, such as the Internet, a local area network (LAN), a wide area network (WAN), a metropolitan area network (MAN), and/or the like, via suitable wired and wireless networking connections.

Depending on the implementation, the one or more health-monitoring data-sources 506 may comprise one or more personalized health-monitoring or health-data-acquisition devices such as wearable health-monitoring devices 506A (e.g., smartwatches) for collecting patients' physiological data (such as heart rates, heart rhythms, blood pressures, breathing patterns, blood glucose levels, and/or the like), analytical health-monitoring devices and system 506B such as the portable electrochemical-sensor system 100 described above, for analyzing patients' biological samples (e.g., bodily fluid, tissue, and/or the like), portable health-monitoring devices (e.g., portable blood-pressure monitors), and/or similar devices.

The one or more health-monitoring data-sources 506 may alternatively or may also comprise one or more medical records such as medication records 506C collected by patients' and/or doctors' computing devices, medical imaging records 506D collected by medical devices of hospitals and/or medical labs, test-result records 506E such as blood test results conducted by hospitals and/or medical labs, and/or the like. Such computing devices and medical devices for obtaining the medical records 506C to 506E may be part of the system 500 in some embodiments. In some other embodiments, the computing devices and medical devices for obtaining the medical records 506C to 506E may not be part of the system 500. Rather, the system 500 provides a data-source interface for interacting with these computing devices and medical devices and receiving medication records 506C to 506E therefrom.

The server computer 502 executes one or more server programs. Depending on implementation, the server computer 502 may be a server-computing device, and/or a general-purpose computing device acting as a server computer while also being used by a user.

The client-computing devices 504 include one or more client-computing devices 504A used by one or more patients and one or more client-computing devices 504B used by doctors. Each client-computing device 504 executes one or more client application programs (or so-called "Apps") and for users to use. The client-computing devices 504 may be portable computing devices such as laptop computers, tablets, smartphones, Personal Digital Assistants (PDAs) and the like. However, those skilled in the art will appreciate that one or more client-computing devices 504 may be non-portable computing devices such as desktop computers in some alternative embodiments.

Generally, the computing devices 502 and 504 have a similar hardware structure such as a hardware structure 520 shown in FIG. 8. As shown, the computing device 502/504 comprises a processing structure 522, a controlling structure 524, memory or storage 526, a networking interface 528, coordinate input 530, display output 532, and other input and output modules 534 and 536, all functionally interconnected by a system bus 538.

The processing structure 522 may be one or more single-core or multiple-core computing processors such as INTEL^{®} microprocessors (INTEL is a registered trademark of Intel Corp., Santa Clara, CA, USA), AMD^{®} microprocessors (AMD is a registered trademark of Advanced Micro Devices Inc., Sunnyvale, CA, USA), ARM^{®} microprocessors (ARM is a registered trademark of Arm Ltd., Cambridge, UK) manufactured by a variety of manufactures such as Qualcomm of San Diego, California, USA, under the ARM^{®} architecture, or the like.

The controlling structure 524 comprises one or more controlling circuits, such as graphic controllers, input/output chipsets and the like, for coordinating operations of various hardware components and modules of the computing device 502/504.

The memory 526 comprises a plurality of memory units accessible by the processing structure 522 and the controlling structure 524 for reading and/or storing data, including input data and data generated by the processing structure 522 and the controlling structure 524. The memory 526 may be volatile and/or non-volatile, non-removable or removable memory such as RAM, ROM, EEPROM, solid-state memory, hard disks, CD, DVD, flash memory, or the like. In use, the memory 526 is generally divided to a plurality of portions for different use purposes. For example, a portion of the memory 526 (denoted as storage memory herein) may be used for long-term data storing, for example, storing files or databases. Another portion of the memory 526 may be used as the system memory for storing data during processing (denoted as working memory herein).

The networking interface 528 comprises one or more networking modules for connecting to other computing devices or networks through the network 508 by using suitable wired or wireless communication technologies.

The display output 532 comprises one or more display modules for displaying images, such as monitors, LCD displays, LED displays, projectors, and the like. The display output 532 may be a physically integrated part of the computing device 502/504 (for example, the display of a laptop computer or tablet), or may be a display device physically separate from, but functionally coupled to, other components of the computing device 502/504 (for example, the monitor of a desktop computer).

The coordinate input 530 comprises one or more input modules for one or more users to input coordinate data, such as touch-sensitive screen, touch-sensitive whiteboard, trackball, computer mouse, touch-pad, or other human interface devices (HID) and the like. The coordinate input 530 may be a physically integrated part of the computing device 502/504 (for example, the touch-pad of a laptop computer or the touch-sensitive screen of a tablet), or may be a display device physically separate from, but functionally coupled to, other components of the computing device 502/504 (for example, a computer mouse). The coordinate input 530, in some implementation, may be integrated with the display output 532 to form a touch-sensitive screen or touch-sensitive whiteboard.

The computing device 502/504 may also comprise other input 534 such as keyboards, microphones, scanners, cameras, Global Positioning System (GPS) component, and/or the like. The computing device 502/504 may further comprise other output 536 such as speakers, printers and/or the like.

The system bus 538 interconnects various components 522 to 536 enabling them to transmit and receive data and control signals to/from each other.

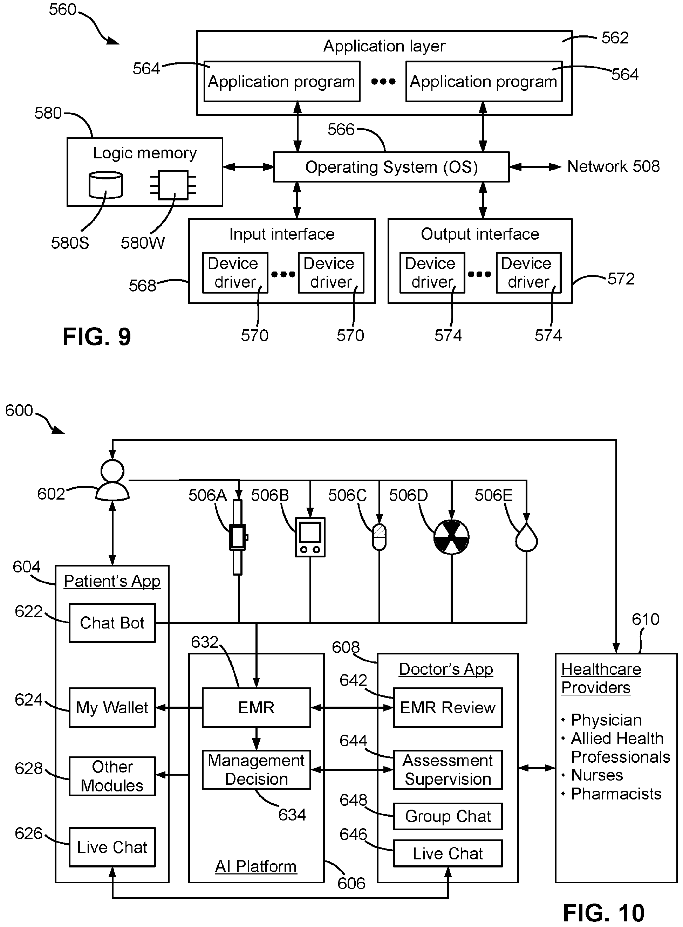

FIG. 9 shows a simplified software architecture 560 of the computing device 502 or 504. The software architecture 560 comprises an application layer 562, an operating system 566, an input interface 568, an output interface 572, and logic memory 580. The application layer 562 comprises one or more application programs 564 executed by or run by the processing structure 522 for performing various tasks. The operating system 566 manages various hardware components of the computing device 502 or 504 via the input interface 568 and the output interface 572, manages logic memory 580, and manages and supports the application programs 564. The operating system 566 is also in communication with other computing devices (not shown) via the network 508 to allow application programs 564 to communicate with those running on other computing devices. As those skilled in the art will appreciate, the operating system 566 may be any suitable operating system such as MICROSOFT^{®} WINDOWS^{®} (MICROSOFT and WINDOWS are registered trademarks of the Microsoft Corp., Redmond, WA, USA), APPLE^{®} OS X, APPLE^{®} iOS (APPLE is a registered trademark of Apple Inc., Cupertino, CA, USA), Linux, ANDROID^{®} (ANDROID is a registered trademark of Google Inc., Mountain View, CA, USA), or the like. The computing devices 502 and 504 of the personalized health-monitoring system 500 may all have the same operating system, or may have different operating systems.

The input interface 568 comprises one or more input device drivers 570 for communicating with respective input devices including the coordinate input 530. The output interface 572 comprises one or more output device drivers 574 managed by the operating system 566 for communicating with respective output devices including the display output 532. Input data received from the input devices via the input interface 568 is sent to the application layer 562, and is processed by one or more application programs 564. The output generated by the application programs 564 is sent to respective output devices via the output interface 572.

The logical memory 580 is a logical mapping of the physical memory 526 for facilitating the application programs 564 to access. In this embodiment, the logical memory 580 comprises a storage memory area (580S) that is usually mapped to non-volatile physical memory such as hard disks, solid state disks, flash drives, and the like, generally for long-term data storage therein. The logical memory 580 also comprises a working memory area (580W) that is generally mapped to high-speed, and in some implementations volatile, physical memory such as RAM, generally for application programs 564 to temporarily store data during program execution. For example, an application program 564 may load data from the storage memory area 580S into the working memory area 580W, and may store data generated during its execution into the working memory area 580W. The application program 564 may also store some data into the storage memory area 580S as required or in response to a user's command.

In a server computer 502, the application layer 562 generally comprises one or more server-side application programs 564 which provide server functions for managing network communication with client-computing devices 504 and facilitating collaboration between the server computer 502 and the client-computing devices 504. Herein, the term "server" may refer to a server computer 502 from a hardware point of view or a logical server from a software point of view, depending on the context.

FIG. 10 is a block diagram showing a functional structure 600 of the personalized health-monitoring system 500. As shown, the functional structure 600 of the personalized health-monitoring system 500 comprises a patient's app 604 which is a client-side application program 564 running on a client-computing device 504A of the patient 602, a cloud-based health-monitoring platform 606 using artificial intelligence (AI) and comprising a set of server-side application programs 564 running on a server computer 502, and a doctor's app 608 which is a client-side application program 564 running on a client-computing device 504B of the doctor (not shown).

In these embodiments, the personalized health-monitoring system 500 is a subscription-based system to patients 602. Accordingly, the patient's app 604 comprises necessary function modules (not shown) for subscribing the service of the system 500, logging-in, authentication, and the like. The patient's App 604 also comprises a user-interaction module (denoted as a "ChatBot") 622, a secured personal-data storage (denoted as "My Wallet") 624, a live-chat module 626, and other modules 628.

The user-interaction module 622 provides the patient 602 a customized questionnaire comprising a plurality of health survey-questions for surveying the general health conditions and life style of the patient 602. The patient may go through the questionnaire and provide their answer to the health survey questions thereof. The system 500 repeatedly update the questionnaire to adapt the questionnaire to the patient's particular health conditions.

The secured personal-data storage 624 in these embodiments is a database with enhanced security for safely storing the patient's personal and health data.

As will be described later, the live-chat module 626 establishes and maintains real-time multimedia communication (e.g., in text, image, voice, and video) with the doctor's App 608 of the patient's doctor. The live-chat modules 626 and 646 may also comprise a suitable file-transfer function for transferring files therebetween. In some embodiments, the live-chat module 626 also provides a group-chat function allowing a plurality of patients 602 to use their patient's Apps 604 to chat with one or more doctors via the doctor's Apps 608. In some embodiments, the group-chat function may be implemented as a separate function module.

The patient's App 604 also comprises other modules 628 as needed. For example, in some embodiments, the other modules 628 may include a medication-list module for the patient to submit their prescribed and/or current medication list to the doctor, a self-management module for the patient to manage their health-related items such as activities, diets, and the like and also to manage the settings of the patient's App 604, and a news feed module for sending, sharing, receiving, and saving news and posts.

The health-monitoring platform 606 comprises a health database 632 for storing the personal and health data of the patient 602 as electronic medical records (EMRs) and a management and decision module 634. The management and decision module 634 automatically analyzes the health data (collected by the health-monitoring data-sources 506 and stored in the health database 632 as EMRs) and assess the health conditions of the one or more patients 602 for obtaining health-analysis results and corresponding health and medical advices or suggestions.

The doctor's App 608 comprises an EMR review module 642 for reviewing the health data of the patient 602 stored in the health database 632, an assessment supervision module 644 for reviewing, modifying, confirming, and/or forming personalized health and/or medical advices, a live-chat module 646 for communicating with the live-chat module 626 of the patient's App 604, and other modules 648 such as a module for staff management. In these embodiments the doctor's App 608 also comprises an interface (not shown) for communicating and interacting with healthcare providers 610 such as other physicians, allied health professionals, pharmacists, and/or the like, to facilitate the healthcare providers 610 in providing services to the patient 602.

In these embodiments, the EMR review module 642 may receive the doctor's input and instruct the server 502 to revise the health-analysis results and corresponding health and medical advices or suggestions obtained by the management and decision module 634.

In some embodiments, the live-chat module 646 may also provide a group-chat function allowing the doctor to chat with a plurality of patients. In some embodiments, the live-chat module 646 may further allow group chat with other users such as between doctors, between doctors and staff, between staff members, between doctors and other health providers, and/or the like. In some embodiments, the group-chat function with other users may be controlled by the user/doctor of the doctor's App 608 and/or the system policy settings such that the ability of initiating a group chat from certain users is restricted. For example, in some embodiments, a group chat between doctors and staff members may be initiated by a doctor, but is not allowed to be initiated by a staff member.

With the functional structure 600 shown in FIG. 10, the user-interaction module collects answers of the questionnaire from the patient 602 and sends collected questionnaire answers to the health-monitoring platform 606 for establishing or updating the patient's personal and health data. Specifically, the health-monitoring platform 606 associates the received health data with the patient 602 and stores received health data in the health database 632 as EMRs.

The one or more health-monitoring data-sources 506 such as the wearable health-monitoring devices 506A, analytical health-monitoring devices 506B, medication records 506C, medical imaging records 506D, test-result records 506E, and the like, also transmit health data of the patient 602 to the health-monitoring platform 606. The health-monitoring platform 606 associates the received health data with the patient 602 and stores received health data in the health database 632 as EMRs.

After the EMRs of the patient 602 are established or updated, the EMRs are also sent to the patient's App 604 and stored in the secured storage thereof. A notification may also be sent to the patient's App 604 to notify the patient that his/her health data has been updated.

When the patient's health data is updated or upon a request from the patient's doctor, the health-monitoring platform 606 also sends the EMRs of the patient 602 to the EMR review module 642 of the doctor's App used by the patient's doctor.

The management decision module 634 of the health-monitoring platform 606 automatically accesses the health database 632 and uses a suitable AI method to analyze the health data (i.e., EMRs) of the patient 602 to determine the patient's health conditions and corresponding health and medical advices or suggestions. The analytical results and advices (collectively denoted as "health results" hereinafter) determined by the management decision module 634 are sent to the doctor's App 608 such that the doctor may use the assessment supervision module 644 of the doctor's App 608 to review, modify, confirm, and/or form personalized health and/or medical advices. When needed, the doctor may use the assessment supervision module 644 of the doctor's App 608 to query the management decision module 634 for reviewing, modifying, confirming, and/or forming personalized health and/or medical advices.

In these embodiments, the health results (in which the advices may be modified by the patient's doctor as described above) are not directly sent to the patent's App 604. Rather, the doctor and the patient 602 may use their live-chat modules 646 and 626 of the doctor's App 608 and the patient's App 604, respective to communicate with respect to the health results. The doctor may use the file-transfer function of the live-chat module 646 to send the health results or a portion thereof to the patient 602. Therefore, the patient 602 does not need to visit the doctor's office for obtaining the health results.

In these embodiments, a live chat may only be initiated by the live-chat module 646 of the doctor's App 608. Of course, those skilled in the art will appreciate that in some alternative embodiments, the live-chat module 626 of the patient's App 608 may also initiate a live chat with the live-chat module 646 of the doctor's App 608.

For example, if a patient is tested positive as having been infected by COVID-19 or similar type of contagious infections, the App may notify the patent and/or the central healthcare system. The central healthcare system may contact the patient for a second confirmatory test. Upon the confirmation of the second test, if the patient does not need the care in hospital, they may self-isolate at home and use the serological kits to monitor their recovery.

All of the initial diagnostic kits data and recovery responses of the patient may be monitored in the patient's profile stored in a server. If the quantitative serological response shows the full recovery of the patient based on the server data (e.g., kits results and patient's symptoms collected via the App), the patient may be notified officially that they are fully recovered and may safely come out of self-isolation.

If the quantitative serological trend of each COVID-19 positive patient (e.g., tested every 2-3 days by the patient) does not show full recovery, the patient must remain self-isolated. This comprehensive strategy will be effective for controlling and limiting the outbreak and transmission of the COVID-19-like infection. The data collected from the RNA-diagnostic kits and serological assays along with the symptoms of patients, available in the server, may be used by an authorized organization such as one or more government agencies, to predict the outbreak, identify the source of spread, predict the recovery curve quantitatively, and develop effective strategies for public health management and resuming the businesses.

Although embodiments have been described above with reference to the accompanying drawings, those of skill in the art will appreciate that variations and modifications may be made without departing from the scope thereof as defined by the appended claims.

## Claims

1. A method for detecting an infectious agent, the method comprising:
inserting a distal end of an electrochemical sensing structure (104) into a coupling port (118) of an analyte processing and presentation apparatus (106) for establishing fluid communication between a sampling region (134) at the distal end of the electrochemical sensing structure (104) and one or more microfluidic channels (204) of the analyte processing and presentation apparatus (106) via the coupling port (118) thereof;
applying the bodily fluid sample to the analyte processing and presentation apparatus (106) via a sampling port (120) thereof, the sampling port (120) being in fluid communication with the coupling port (118) via the one or more microfluidic channels (204);
allowing the bodily fluid sample to flow from the sampling port (120) through the one or more microfluidic channels (204) and the coupling port (118) of the analyte processing and presentation apparatus (106) to the sampling region (134) of the electrochemical sensing structure (104); and
coupling a first circuitry of the electrochemical sensing structure (104) with the analysis circuitry of a bio-sensing apparatus (102), the first circuitry of the electrochemical sensing structure (104) comprising a first set of electrodes (124', 126', 128') extending into the sampling region (134) for contacting the bodily fluid sample;
and
detecting, using the analysis circuitry of the bio-sensing apparatus (102), the infectious agent from the bodily fluid sample received in the sampling region (134) of the electrochemical sensing structure (104).

2. The method of claim 1 further comprising:
removing the electrochemical sensing structure (104) from the analyte processing and presentation apparatus (106).

3. A system for detecting an infectious agent, the system comprising a bio-sensing apparatus (102), an electrochemical sensing structure (104) and an analyte processing and presentation apparatus (106);
the bio-sensing apparatus (102) comprising an analysis circuitry for coupling with a first circuitry of the electrochemical sensing structure (104), the first circuitry of the electrochemical sensing structure (104) comprising a first set of electrodes (124', 126', 128') extending into a sampling region (134) at the distal end of the electrochemical sensing structure (104);
the analyte processing and presentation apparatus (106) comprising a sampling port (120) for receiving a bodily fluid sample, and a coupling port (118) in fluid communication with the sampling port (120) via one or more microfluidic channels (204);
**characterised in that** a distal end of the electrochemical sensing structure (104) is adapted for being inserted into the coupling port (118) of the analyte processing and presentation apparatus (106) for establishing fluid communication between the sampling region (134) at the distal end of the electrochemical sensing structure (104) and the one or more microfluidic channels (204) of the analyte processing and presentation apparatus (106), for directing the bodily fluid sample from the sampling port (120) of the analyte processing and presentation apparatus (106) to the sampling region (134) at the distal end of the electrochemical sensing structure (104).

4. The system of claim 3, wherein the electrochemical sensing structure (104) further comprises an identification circuitry separated from the first circuitry for indicating one or more biomarkers of the bodily fluid sample analyzable using the electrochemical-sensor structure.

5. The system of claim 3 or 4, wherein the electrochemical sensing structure (104) is a testing strip.

6. The system of any one of claims 3 to 5, wherein the analyte processing and presentation apparatus (106) is a microcolumn device or a lateral-flow device.

7. The system of any one of claims 3 to 6, wherein the analyte processing and presentation apparatus (106) is a flow-cell device comprising one or more flow cells;
wherein each of the one or more flow cells comprises:
an inlet for receiving the bodily fluid sample from the first port,
an outlet in the second port, and
a flow path for delivering the bodily fluid sample from the inlet to the outlet; and
wherein the flow path comprises a plurality of chambers embossed therealong.

8. The system of claim 7, wherein the plurality of chambers are configured for limiting a flow rate of the bodily fluid sample in the flow path.

9. The system of claim 7 or 8, wherein a first set of one or more of the plurality of chambers comprise a first type of embossed or debossed structures for promoting capillary flow.

10. The system of any one of claims 7 to 9, wherein a second set of one or more of the plurality of chambers comprise embossed or debossed structures for mixing between two or more molecular, oligonucleotide, enzymatic, or protein entities with the bodily fluid sample.

11. The system of any one of claims 7 to 10, wherein a third set of one or more of the plurality of chambers comprise conductive substrates for detecting impedimetric changes caused by the bodily fluid sample.

12. The system of any one of claims 7 to 11, wherein a fourth set of one or more of the plurality of chambers comprise optically active components with changeable absorptive properties correlated to the volume, viscosity, reflectance, and/or fluoro-emissive properties of the bodily fluid sample.

13. The system of any one of claims 7 to 12, wherein the analyte processing and presentation apparatus (106) comprises a plurality of flow cells.

14. The system of claim 13, wherein each flow cell is configured for preparing and presenting the bodily fluid sample with a different degree of dilution.

15. The system of claim 13 or 14, wherein each flow cell is configured for preparing and presenting a portion of the bodily fluid sample to the outlet thereof; and
wherein said preparing and presenting the portion of the bodily fluid sample comprises reacting, mixing, binding, or entrapping the bodily fluid sample with molecules, enzymes, oligonucleotides, and/or antibodies within the flow path of the flow cell.

16. The system of any one of claims 3 to 15 further comprising one or more sample collection devices for using with the analyte processing and presentation apparatus (106) for processing and presenting the bodily fluid sample to the electrochemical-sensor structure.

## Patentansprüche

1. Verfahren zum Detektieren eines Infektionserregers, wobei das Verfahren umfasst:
Einführen eines distalen Endes einer elektrochemischen Erfassungsstruktur (104) in einen Kopplungsanschluss (118) einer Analytverarbeitungs- und -präsentationsvorrichtung (106) zum Herstellen einer Fluidverbindung zwischen einer Probenregion (134) am distalen Ende der elektrochemischen Erfassungsstruktur (104) und einem oder mehreren mikrofluidischen Kanälen (204) der Analytverarbeitungs- und -präsentationsvorrichtung (106) über den Kopplungsanschluss (118) davon;
Aufbringen der Körperflüssigkeitsprobe auf die Analytverarbeitungs- und -präsentationsvorrichtung (106) über einen Probenanschluss (120) davon, wobei der Probenanschluss (120) in Fluidverbindung mit dem Kopplungsanschluss (118) über den einen oder die mehreren mikrofluidischen Kanäle (204) steht;
Ermöglichen, dass die Körperflüssigkeitsprobe vom Probenanschluss (120) durch den einen oder die mehreren mikrofluidischen Kanäle (204) und den Kopplungsanschluss (118) der Analytverarbeitungs- und -präsentationsvorrichtung (106) zur Probenregion (134) der elektrochemischen Erfassungsstruktur (104) fließt; und
Koppeln einer ersten Schaltung der elektrochemischen Erfassungsstruktur (104) mit der Analyseschaltung einer Biosensorvorrichtung (102), wobei die erste Schaltung der elektrochemischen Erfassungsstruktur (104) einen ersten Satz von Elektroden (124', 126', 128') umfasst, die sich in die Probenregion (134) erstrecken, zum Kontaktieren der Körperflüssigkeitsprobe; und
Detektieren, unter Verwendung der Analyseschaltung der Biosensorvorrichtung (102), des Infektionserregers aus der Körperflüssigkeitsprobe, die in der Probenregion (134) der elektrochemischen Erfassungsstruktur (104) empfangen wird.

2. Verfahren nach Anspruch 1, ferner umfassend:
Entfernen der elektrochemischen Erfassungsstruktur (104) von der Analytverarbeitungs- und - präsentationsvorrichtung (106).

3. System zum Detektieren eines Infektionserregers, wobei das System eine Biosensorvorrichtung (102), eine elektrochemische Erfassungsstruktur (104) und eine Analytverarbeitungs- und - präsentationsvorrichtung (106) umfasst;
wobei die Biosensorvorrichtung (102) eine Analyseschaltung zum Koppeln mit einer ersten Schaltung der elektrochemischen Erfassungsstruktur (104) umfasst, wobei die erste Schaltung der elektrochemischen Erfassungsstruktur (104) einen ersten Satz von Elektroden (124', 126', 128') umfasst, die sich in eine Probenregion (134) am distalen Ende der elektrochemischen Erfassungsstruktur (104) erstrecken;
wobei die Analytverarbeitungs- und -präsentationsvorrichtung (106) einen Probenanschluss (120) zum Empfangen einer Körperflüssigkeitsprobe und einen Kopplungsanschluss (118) in Fluidverbindung mit dem Probenanschluss (120) über einen oder mehrere mikrofluidische Kanäle (204) umfasst;
**dadurch gekennzeichnet, dass** ein distales Ende der elektrochemischen Erfassungsstruktur (104) ausgebildet ist zum Einführen in den Kopplungsanschluss (118) der Analytverarbeitungs- und - präsentationsvorrichtung (106) zum Herstellen einer Fluidverbindung zwischen der Probenregion (134) am distalen Ende der elektrochemischen Erfassungsstruktur (104) und dem einen oder den mehreren mikrofluidischen Kanälen (204) der Analytverarbeitungs- und -präsentationsvorrichtung (106), zum Leiten der Körperflüssigkeitsprobe vom Probenanschluss (120) der Analytverarbeitungs- und -präsentationsvorrichtung (106) zur Probenregion (134) am distalen Ende der elektrochemischen Erfassungsstruktur (104).

4. System nach Anspruch 3, wobei die elektrochemische Erfassungsstruktur (104) ferner eine Identifikationsschaltung umfasst, die von der ersten Schaltung getrennt ist, zum Anzeigen eines oder mehrerer Biomarker der Körperflüssigkeitsprobe, die unter Verwendung der elektrochemischen Sensorstruktur analysierbar sind.

5. System nach Anspruch 3 oder 4, wobei die elektrochemische Erfassungsstruktur (104) ein Teststreifen ist.

6. System nach einem der Ansprüche 3 bis 5, wobei die Analytverarbeitungs- und - präsentationsvorrichtung (106) eine Mikrosäulenvorrichtung oder eine Lateral-Flow-Vorrichtung ist.

7. System nach einem der Ansprüche 3 bis 6, wobei die Analytverarbeitungs- und - präsentationsvorrichtung (106) eine Flusszellvorrichtung ist, die eine oder mehrere Flusszellen umfasst;
wobei jede der einen oder mehreren Flusszellen umfasst:
einen Einlass zum Empfangen der Körperflüssigkeitsprobe vom ersten Anschluss,
einen Auslass im zweiten Anschluss, und
einen Fließweg zum Liefern der Körperflüssigkeitsprobe vom Einlass zum Auslass; und
wobei der Fließweg eine Vielzahl von Kammern umfasst, die entlang davon geprägt sind.

8. System nach Anspruch 7, wobei die Vielzahl von Kammern konfiguriert ist zum Begrenzen einer Fließrate der Körperflüssigkeitsprobe im Fließweg.

9. System nach Anspruch 7 oder 8, wobei ein erster Satz von einer oder mehreren der Vielzahl von Kammern eine erste Art von geprägten oder vertieften Strukturen umfasst zum Fördern eines Kapillarflusses.

10. System nach einem der Ansprüche 7 bis 9, wobei ein zweiter Satz von einer oder mehreren der Vielzahl von Kammern geprägte oder vertiefte Strukturen umfasst zum Mischen zwischen zwei oder mehr molekularen, Oligonukleotid-, enzymatischen oder Proteinentitäten mit der Körperflüssigkeitsprobe.

11. System nach einem der Ansprüche 7 bis 10, wobei ein dritter Satz von einer oder mehreren der Vielzahl von Kammern leitfähige Substrate umfasst zum Detektieren impedimetrischer Änderungen, die durch die Körperflüssigkeitsprobe verursacht werden.

12. System nach einem der Ansprüche 7 bis 11, wobei ein vierter Satz von einer oder mehreren der Vielzahl von Kammern optisch aktive Komponenten mit veränderbaren absorptiven Eigenschaften umfasst, die mit dem Volumen, der Viskosität, dem Reflexionsvermögen und/oder den fluoro-emissiven Eigenschaften der Körperflüssigkeitsprobe korreliert sind.

13. System nach einem der Ansprüche 7 bis 12, wobei die Analytverarbeitungs- und - präsentationsvorrichtung (106) eine Vielzahl von Flusszellen umfasst.

14. System nach Anspruch 13, wobei jede Flusszelle konfiguriert ist zum Vorbereiten und Präsentieren der Körperflüssigkeitsprobe mit einem unterschiedlichen Verdünnungsgrad.

15. System nach Anspruch 13 oder 14, wobei jede Flusszelle konfiguriert ist zum Vorbereiten und Präsentieren eines Teils der Körperflüssigkeitsprobe zum Auslass davon; und
wobei das Vorbereiten und Präsentieren des Teils der Körperflüssigkeitsprobe Reagieren, Mischen, Binden oder Einfangen der Körperflüssigkeitsprobe mit Molekülen, Enzymen, Oligonukleotiden und/oder Antikörpern innerhalb des Fließwegs der Flusszelle umfasst.

16. System nach einem der Ansprüche 3 bis 15, ferner umfassend eine oder mehrere Probensammelvorrichtungen zur Verwendung mit der Analytverarbeitungs- und - präsentationsvorrichtung (106) zum Verarbeiten und Präsentieren der Körperflüssigkeitsprobe an die elektrochemische Sensorstruktur.

## Revendications

1. Procédé de détection d'un agent infectieux, le procédé comprenant :
l'insertion d'une extrémité distale d'une structure de détection électrochimique (104) dans un orifice de couplage (118) d'un appareil de traitement et de présentation d'analyte (106) pour établir une communication fluidique entre une région d'échantillonnage (134) au niveau de l'extrémité distale de la structure de détection électrochimique (104) et un ou plusieurs canaux microfluidiques (204) de l'appareil de traitement et de présentation d'analyte (106) par l'intermédiaire de l'orifice de couplage (118) de celui-ci ;
l'application de l'échantillon de fluide corporel à l'appareil de traitement et de présentation d'analyte (106) par l'intermédiaire d'un orifice d'échantillonnage (120) de celui-ci, l'orifice d'échantillonnage (120) étant en communication fluidique avec l'orifice de couplage (118) par l'intermédiaire du ou des canaux microfluidiques (204) ;
le fait de permettre à l'échantillon de fluide corporel de s'écouler depuis l'orifice d'échantillonnage (120) à travers le ou les canaux microfluidiques (204) et l'orifice de couplage (118) de l'appareil de traitement et de présentation d'analyte (106) jusqu'à la région d'échantillonnage (134) de la structure de détection électrochimique (104) ; et
le couplage d'un premier circuit de la structure de détection électrochimique (104) avec le circuit d'analyse d'un appareil de biodétection (102), le premier circuit de la structure de détection électrochimique (104) comprenant un premier ensemble d'électrodes (124', 126', 128') qui s'étendent dans la région d'échantillonnage (134) pour entrer en contact avec l'échantillon de fluide corporel ; et
la détection, en utilisant le circuit d'analyse de l'appareil de biodétection (102), de l'agent infectieux à partir de l'échantillon de fluide corporel reçu dans la région d'échantillonnage (134) de la structure de détection électrochimique (104).

2. Procédé selon la revendication 1, comprenant en outre :
le retrait de la structure de détection électrochimique (104) de l'appareil de traitement et de présentation d'analyte (106).

3. Système de détection d'un agent infectieux, le système comprenant un appareil de biodétection (102), une structure de détection électrochimique (104) et un appareil de traitement et de présentation d'analyte (106) ;
l'appareil de biodétection (102) comprenant un circuit d'analyse pour le couplage avec un premier circuit de la structure de détection électrochimique (104), le premier circuit de la structure de détection électrochimique (104) comprenant un premier ensemble d'électrodes (124', 126', 128') qui s'étendent dans une région d'échantillonnage (134) au niveau de l'extrémité distale de la structure de détection électrochimique (104) ;
l'appareil de traitement et de présentation d'analyte (106) comprenant un orifice d'échantillonnage (120) pour recevoir un échantillon de fluide corporel, et un orifice de couplage (118) en communication fluidique avec l'orifice d'échantillonnage (120) par l'intermédiaire d'un ou plusieurs canaux microfluidiques (204) ;
**caractérisé en ce qu'**une extrémité distale de la structure de détection électrochimique (104) est adaptée pour être insérée dans l'orifice de couplage (118) de l'appareil de traitement et de présentation d'analyte (106) pour établir une communication fluidique entre la région d'échantillonnage (134) au niveau de l'extrémité distale de la structure de détection électrochimique (104) et le ou les canaux microfluidiques (204) de l'appareil de traitement et de présentation d'analyte (106), pour diriger l'échantillon de fluide corporel depuis l'orifice d'échantillonnage (120) de l'appareil de traitement et de présentation d'analyte (106) jusqu'à la région d'échantillonnage (134) au niveau de l'extrémité distale de la structure de détection électrochimique (104).

4. Système selon la revendication 3, dans lequel la structure de détection électrochimique (104) comprend en outre un circuit d'identification qui est séparé du premier circuit pour indiquer un ou plusieurs biomarqueurs de l'échantillon de fluide corporel analysable en utilisant la structure de capteur électrochimique.

5. Système selon la revendication 3 ou 4, dans lequel la structure de détection électrochimique (104) est une bandelette de test.

6. Système selon l'une quelconque des revendications 3 à 5, dans lequel l'appareil de traitement et de présentation d'analyte (106) est un dispositif à microcolonne ou un dispositif à flux latéral.

7. Système selon l'une quelconque des revendications 3 à 6, dans lequel l'appareil de traitement et de présentation d'analyte (106) est un dispositif à cellule d'écoulement comprenant une ou plusieurs cellules d'écoulement ;
dans lequel chacune de la ou des cellules d'écoulement comprend :
une entrée pour recevoir l'échantillon de fluide corporel depuis le premier orifice,
une sortie dans le second orifice, et
un trajet d'écoulement pour délivrer l'échantillon de fluide corporel depuis l'entrée jusqu'à la sortie ; et
dans lequel le trajet d'écoulement comprend une pluralité de chambres qui sont en relief le long de celui-ci.

8. Système selon la revendication 7, dans lequel la pluralité de chambres sont configurées pour limiter un débit de l'échantillon de fluide corporel dans le trajet d'écoulement.

9. Système selon la revendication 7 ou 8, dans lequel un premier ensemble d'une ou plusieurs de la pluralité de chambres comprend un premier type de structures en relief ou en creux pour favoriser l'écoulement capillaire.

10. Système selon l'une quelconque des revendications 7 à 9, dans lequel un second ensemble d'une ou plusieurs de la pluralité de chambres comprend des structures en relief ou en creux pour le mélange entre deux ou plusieurs entités moléculaires, oligonucléotidiques, enzymatiques ou protéiques avec l'échantillon de fluide corporel.

11. Système selon l'une quelconque des revendications 7 à 10, dans lequel un troisième ensemble d'une ou plusieurs de la pluralité de chambres comprend des substrats conducteurs pour détecter des changements impédimétriques causés par l'échantillon de fluide corporel.

12. Système selon l'une quelconque des revendications 7 à 11, dans lequel un quatrième ensemble d'une ou plusieurs de la pluralité de chambres comprend des composants optiquement actifs avec des propriétés d'absorption changeables qui sont corrélées au volume, à la viscosité, à la réflectance et/ou aux propriétés fluoro-émissives de l'échantillon de fluide corporel.

13. Système selon l'une quelconque des revendications 7 à 12, dans lequel l'appareil de traitement et de présentation d'analyte (106) comprend une pluralité de cellules d'écoulement.

14. Système selon la revendication 13, dans lequel chaque cellule d'écoulement est configurée pour préparer et présenter l'échantillon de fluide corporel avec un degré différent de dilution.

15. Système selon la revendication 13 ou 14, dans lequel chaque cellule d'écoulement est configurée pour préparer et présenter une portion de l'échantillon de fluide corporel à la sortie de celle-ci ; et
dans lequel ladite préparation et présentation de la portion de l'échantillon de fluide corporel comprend la réaction, le mélange, la liaison ou le piégeage de l'échantillon de fluide corporel avec des molécules, des enzymes, des oligonucléotides et/ou des anticorps à l'intérieur du trajet d'écoulement de la cellule d'écoulement.

16. Système selon l'une quelconque des revendications 3 à 15, comprenant en outre un ou plusieurs dispositifs de collecte d'échantillon pour une utilisation avec l'appareil de traitement et de présentation d'analyte (106) pour traiter et présenter l'échantillon de fluide corporel à la structure de capteur électrochimique.
